(19) Europäisches Patentamt
European Patent Office
Office européen des brevets

(11) EP 2 708 232 B1

(12) **EUROPEAN PATENT SPECIFICATION**

(45) Date of publication and mention of the grant of the patent:
**09.11.2016 Bulletin 2016/45**

(21) Application number: **12382352.8**

(22) Date of filing: **14.09.2012**

(51) Int Cl.:
*A61K 36/185* (2006.01) *A61K 36/23* (2006.01)
*A61K 36/28* (2006.01) *A61Q 19/00* (2006.01)
*C12N 5/00* (2006.01) *C12N 5/04* (2006.01)

(54) **Composition comprising a Centella asiatica plant suspension cell culture**

Zusammensetzung beinhaltend eine Centella asiatica Pflanzenzellensuspensionskultur

Composition comprenant une culture en suspension de Centella asiatica

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.03.2014 Bulletin 2014/12**

(73) Proprietor: **Casen Recordati, S.L.**
**50180 Utebo (Zaragoza) (ES)**

(72) Inventors:
- **Tabuenca Navarro, Daniel.**
  **50180 UTEBO (ES)**
- **Bello Navarro, Marta.**
  **50180 UTEBO (ES)**

(74) Representative: **ZBM Patents - Zea, Barlocci & Markvardsen**
**Plaza Catalunya, 1**
**08002 Barcelona (ES)**

(56) References cited:
- **SUSANA MANGAS ET AL: "Triterpenoid saponin content and the expression level of some related genes in calli of Centella asiatica", BIOTECHNOLOGY LETTERS, SPRINGER NETHERLANDS, DORDRECHT, vol. 30, no. 10, 25 June 2008 (2008-06-25) , pages 1853-1859, XP019600034, ISSN: 1573-6776**
- **S. Mangas ET AL: "Centella asiatica (L) Urban: An updated approach" In: "Plant Secondary Terpenoids", 1 January 2009 (2009-01-01), XP055044432, ISBN: 978-8-13-080332-6 pages 1-20, * the whole document * * ***
- **MERCEDES BONFILL ET AL: "Production of centellosides and phytosterols in cell suspension cultures of", PLANT CELL, TISSUE AND ORGAN CULTURE, KLUWER ACADEMIC PUBLISHERS, DO, vol. 104, no. 1, 9 August 2010 (2010-08-09), pages 61-67, XP019866351, ISSN: 1573-5044, DOI: 10.1007/S11240-010-9804-7**
- **Mangas, S. A.: "Producción de saponinas triterpénicas en cultivos in vitro de Centella asiatica", , 2009, page 13PP, i-v,7,9-45, 47-217, 59PP, XP002687700, Retrieved from the Internet: URL:http://tdx.cesca.cat/handle/10803/2622 [retrieved on 2012-11-16]**
- **anonymous: "Madecassoside 90% - Guangxi Changzhou Natural Pharmaceutical Co., Ltd", , 2011, page 1, XP002687366, Retrieved from the Internet: URL:http://www.changzhou-centella.com/ProductShow.aspx?MyID=5 [retrieved on 2012-11-16]**
- **DATABASE GNPD [Online] MINTEL; November 2011 (2011-11), anonymous: "Medicated Blemish Balm Cream", XP002687367, retrieved from www.gnpd.com Database accession no. 1430228**
- **DATABASE GNPD [Online] MINTEL; December 2010 (2010-12), anonymous: "Stimulant II Corrective Serum", XP002687368, retrieved from www.gnpd.com accession no. 1469715**
- **DATABASE GNPD [Online] MINTEL; October 2009 (2009-10), anonymous: "Ultra Nourishing Legs Cream", XP002687369, retrieved from www.gnpd.com Database accession no. 1202437**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 708 232 B1

- ROZITA OMAR ET AL: "Development of Growth Medium for Centella Asiatica Cell Culture Via Response Surface Methodology", AMERICAN JOURNAL OF APPLIED SCIENCES, vol. 1, no. 3, 1 March 2004 (2004-03-01), pages 215-219, XP055044968, ISSN: 1546-9239, DOI: 10.3844/ajassp.2004.215.219

## Description

The present invention relates to the field of pharmacy, medical devices and cosmetics, in particular to compositions comprising a plant cell culture from a plant of the genus *Centella.* It also relates to methods for obtaining said compositions, and to uses of these compositions as tissue repair agents.

BACKGROUND ART

There exist several compositions including extracts from plants of the *Centella* genus, namely from *Centella asiatica* specie (C. *asiatica*), also named Gotu Kola, Tiger grass and Indian pennywort. The extracts of this plant have been widely used in the management of dermatological conditions, to treat wounds, scratches, superficial burns, eczemas, or as antiinflammatory agent.

The extended use of *Centella asiatica* extracts in regenerating skin compositions, as well as in damaged tissue repairing preparations, is due to the capability of some of the compounds in the plant, which are able to promote collagen synthesis. Some of these active constituents of *Centella asiatica* are pentacyclic triterpenoids, found as genins (asiatic and madecassic acid) and heterosides (asiaticoside and madecassoside) in the plant. Since these compounds are associated with *Centella,* they are also known as centellosides. Maquart et al., "Stimulation of collagen synthesis in fibroblast cultures by a triterpene extracted from Centella asiatica". Conn. Tissue Res -1990, vol. 24, pp. 107-120, disclose indeed that asiaticoside, asiatic and madecassic acid promote collagen synthesis in a dose-dependant manner.

Bonfill et al., 2010, Plant cell, tissue and organ culture, 104(1), 61-67 disclose a Centella asiatica cell suspension method of elicitation of centellosides employing a single addition of methyl jasmonate (MeJA), resulting in madecassoside and asiaticoside present in similar proportions. The would-healing properties of centellosides are disclosed to be known.

Centellosides are a type of secondary metabolites present in *Centella asiatica,* and they are considered to be involved in the response to stress stimuli. Secondary metabolites are compounds synthesized by plant cells, and although not having an apparent function in the plant's primary metabolism, they play important secondary roles, for example by acting as insect repellants, defense molecules against microorganisms, or against external stimuli (cut of some parts of the plant, presence of poisons, etc.). Some of these plant-derived compounds are useful as active pharmacological ingredients. Nonetheless, the chemical synthesis of the same is sometimes difficult and for this reason they are extracted from the plants, and further concentrated and purified if required.

Examples of extracts of *Centella asiatica* include aqueous extracts, alcoholic extracts, and extracts with other solvents. Extraction is usually performed from different parts of the plant including leaves, stems, fruits, seeds, branches, etc.

Titrated Extract of *Centella asiatica* (TECA) is one of the mostly known extracts of this plant. It includes asiaticoside (40 % by weight), and asiatic and madecassic acid (60 % by weight). Madecassoside is not present, since due to the extraction process and due to the high solubility in water, it is eliminated during manipulation. The TECA is thus a standardized extract of *Centella asiatica,* in which the active ingredients are combined in constant proportions to guarantee optimal activity.

On the other hand, the patent document EP867447, filed by Dong Kook Pharmacuetical discloses an hydrosoluble extract including asiaticoside and madecassoside, wherein said asiaticoside and madecassoside are in a ratio ranging from 4:6 to 6:4, both components constituting at least 97 % by weight or more of the extract.

Extraction processes, though standardized, carry out the problem that become quite expensive, especially when the aim is a composition enriched in one specific compound or group of compounds of interest from the selected plant.

An alternative way to obtain a compound of interest from a plant acting as factory is by using the technology of plant cell cultures. Plant cell cultures allow the isolation of cells from different parts of the plant (leaves, fruits, stem, roots, shoots, etc.), which parts of the plant are further cultured under controlled conditions in order to get a desired compound. With the technology of plant cell cultures, isolated plant cells are generally submitted to cell undifferentiation until totipotency is reached. These undifferentiated totipotent cells can then become any plant cell tissue upon adequate stimulation. It is for this reason that these undifferentiated cells are also named plant stem cells.

These totipotent plant cells are used as factories of metabolites or compounds of interest once they are stimulated with specific compounds (also known as eliciting agents). This technology is exemplified in the document of Mulabagal et al., "Plant cell cultures-an alternative and efficient source for the production of biologically important secondary metabolites", International Journal of Applied Science, Engineering and Technology-2004, Vol. 2, pp.: 101-153. Mulabagal et al. disclose callus and suspension culture methods for the production of bioactive secondary metabolites from medicinal plants.

The principle advantage of the technology of plant cell cultures is that it allows the provision of a continuous and reliable source of plant-derived pharmaceuticals.

In the particular case of *Centella asiatica,* a suspension of undifferentiated totipotent cells have been used to

obtain phytosterols and centellosides. The procedure is disclosed in Bonfill et al., "Production of centellosides and phytosterols in cell suspension cultures of Centella asiatica", Plant Cell Tiss Organ Cult - 2011, Vol. No. 104, pp.: 61-67. In this document C. *asiatica* cell suspensions were established and treated with two concentrations (100 and 200 μM) of the eliciting agent (stimulator) methyl jasmonate (MeJA). The maximum centelloside production was observed in the stationary growth phase, reaching 0.16 mg/g of dry weight at day 25 of the culture in the control and 1.11 mg/g at day 15 in the MeJA-elicited cultures. By means of this methodology a percentage by weight of 54.5 % of madecassoside and of 45.5 % of asiaticoside in respect of the total amount of centellosides was obtained.

Although all known compositions comprising components of *Centella asiatica* (mainly extracts of the plant) have interesting properties in terms of promoting tissue repair, especially dermatological tissue, there is still a need of alternative compositions with high capability of inducing collagen synthesis.

SUMMARY OF THE INVENTION

The invention is as set forth in the independent claims. All other embodiments are described for illustrative purposes only, and any statement that they would also be part of the invention or other, similar statements, have to be seen in the context of the application as filed and do not alter the scope of the claims.

It is herewith provided a composition, which can be a pharmaceutical, cosmetic or medical device composition, which has good properties as collagen synthesis inducing agent, and may be used as tissue repair agent or as skin cosmetic care agent, said composition comprising an effective amount of the compound madecassoside, said amount of madecassoside provided by a preparation from a plant of the *Centella* genus.

In a first aspect, thus, the invention relates to a composition comprising an effective amount of a plant cell culture from a plant of the *Centella* genus, said plant cell culture comprising an amount of madecassoside comprised from 75 % to 95 % by weight in respect of the total weight of centellosides in the cell culture.

The repair of the damaged tissues is the result, as will be illustrated in the examples below, of the plant cell culture from a plant of the *Centella* genus with the specific amount of madecassoside. In addition, other of the components of the composition act together with the plant cell culture, and aid to promote also this effect.

The cosmetic effect as skin care agent is also due to the a plant cell culture from a plant of the *Centella* genus and can be improved with other ingredients, phytosterols, flavonoids, natural cellular antioxidants, etc.

Major centellosides are, as above indicated, the genins asiatic and madecassic acid, and also the heterosides asiaticoside and madecassoside, the four represented in formulas (I) to (IV) below:

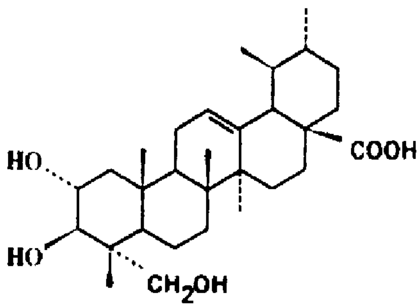

(I) Asiatic acid

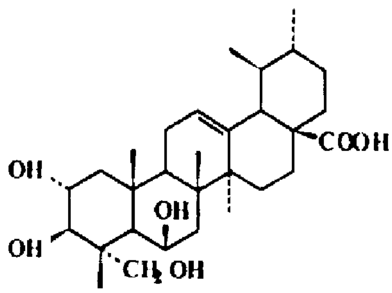

(II) Madecassic acid

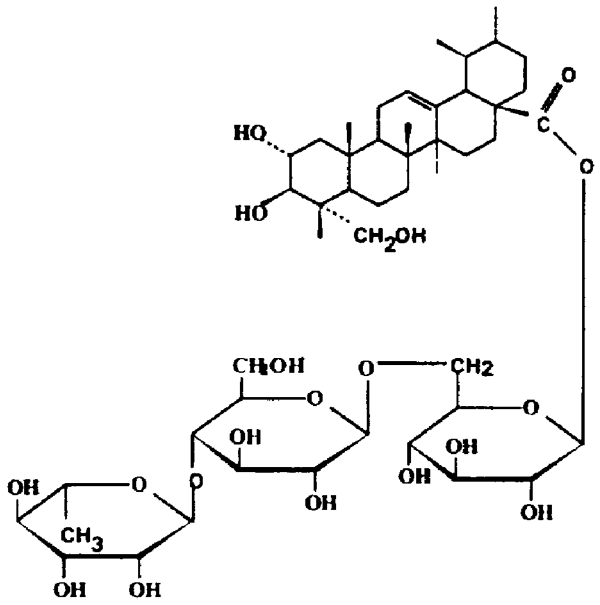

(III) Asiaticoside

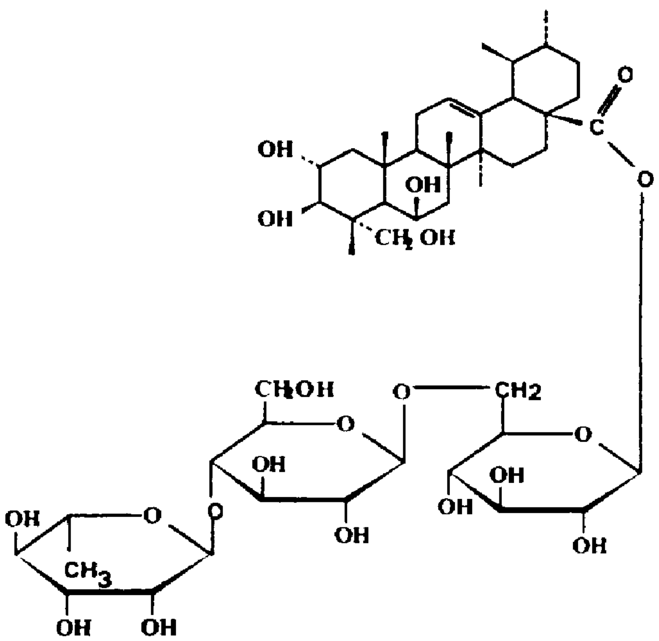

(IV) Madecassoside

For several years it has been believed that asiaticoside was the major component of the centellosides derived from the plants of *Centella* genus having high abilities to restore damaged tissues, such as epidermis, due to its effects as inducer of the collagen synthesis. Nonetheless, the inventors propose the use of other centelloside, namely madecassoside as tissue repair agent.

Another aspect of the present invention is a medical device comprising the composition as defined above. In another aspect, the invention relates to the composition for use as a medical device.

A medical device allows the application of the composition to a desired tissue surface in order to cosmetically, pharmaceutically and/or via the medical device itself treat or take care of the same.

Another aspect of the present invention refers to the use of a topical cosmetic composition or of the medical

device as defined above as a skin care agent, where the skin care comprises ameliorating at least one of the following symptoms: roughness, dehydration, tightness, and lack of elasticity.

All the conditions, which can be treated with the composition of the invention, cosmetically, pharmaceutically and/or via a medical device, may be caused due to surgery interventions, to other pathologies carrying the lost of collagen synthesis by the cells as secondary symptom (stress, chemo and radiotherapeutic treatments, etc.), as well as due to aging, in particular skin aging.

BRIEF DESCRIPTION OF THE DRAWINGS

FIG. 1, related to Example 3, is a bar diagram showing the amount of collagen (μg/$10^6$ human fibroblast cells) synthesized by human fibroblasts after the addition, in the culture medium, of different compositions according to the invention including the plant cell culture of the *Centella* genus.

FIG. 2, related to Example 1, is another graphic with bars showing the amount (concentration in mg/L) of madecassoside (M) and of asiaticoside (A) obtained in a cell suspension of a plant of the *Centella* genus submitted to the method according to the invention. T means treatment or addition of methyl jasmonate; 1X means one addition of methyl jasmonate; 3X means three addition of methyl jasmonate.

DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition. The following definitions are included for the purpose of understanding.

The term "enriched" means herewith that madecassoside is the major component of the total of centellosides produced by the *Centella* genus plant cell culture, namely representing at least 75 % by weight of the total of centellosides (asiaticoside, madecassic acid and asiatic acid) in the plant cell culture. The amount in percentage by weight of madecassoside in the cell culture is, in fact, the representation of the amount in any individual cell constituting said culture, any cell contributing to the final absolute amount of the compound.

The amount of centellosides is preferably determined in the plant cell culture following the HPLC methodology. Cells are lyophilised and extracted through methanol:water extraction, and then dissolved in a polar solvent to perform the HPLC analysis.

In the sense of the present invention a "plant cell culture" are plant cells derived from tissue or cell of plants, which are then cultured in a container or recipient. The plant cell cultures include callus tissues (callus), differentiated cultured tissues or a cultured organ tissue. The callus are, in turn, cultured plant cells aggregates consisting of undifferentiated cells (mass of undifferentiated plant cells) propagated over solid culture medium containing plant hormones or in liquid culture medium containing the plant hormones. On the other hand, the differentiated cultured tissues are aggregates of plant cells constituing a root, a stem, a bud, or a fruit.

Plant "callus" is a mass of unorganized parenchymatic cells derived from plant tissue (explants) for use in biological research and biotechnology. In plant biology, callus cells are those cells that cover a plant wound. Callus formation is induced from plant tissues after surface sterilization and plating onto in vitro tissue culture medium. Plant growth regulators, such as auxins, cytokinins, and gibberellins, are supplemented into the medium to initiate callus formation or somatic embryogenesis.

A "plant cell culture lysate" is a plant cell culture which has been submitted to disaggregation and disruption of the cells, by means of sonication or mechanically, via a cutting-agitator. With the disruption of the cells the content of the cytoplasm is delivered in the medium (solid or liquid), thus leading to a mixture that comprises the different components of the cell (compounds of the cytoplasm, membrane fragments and compounds from the cell previously delivered in the medium). The plant cell culture lysate can also be obtained by other means including, as a non-limitative example, freezing at very low temperature.

In the sense of the invention a "cell suspension culture" or "cell suspension" is a group of undifferentiated cells dispersed or in suspension in a liquid nutrient medium (liquid culture medium). The cell suspension may comprise cells in various stages of aggregation.

In the sense of the invention an "in-stationary phase culture" or "growing culture" used herewith interchangeable, means a culture of cells (undifferentiated or differentiated) from a plant of the *Centella* genus that is grown until a desired phase, in which phase the density of the cell culture is maintained at a constant value because cells neither die nor grow. To this culture further treatments can be applied.

The term "jasmonate derivative compound" or "jasmonate compound", used herewith interchangeable, encompasses compounds derived from jasmonic acid of formula (A), in which one or several hydrogen atoms, namely the hydrogen atom of the hydroxyl compound, have been substituted by a ($C_1$-$C_3$)-alkyl radical. Examples of ($C_1$-$C_3$)-alkyl radical include methyl, ethyl, and propyl. Thus, some of the jasmonate derivatives compounds are methyl jasmonate (formula (B)), ethyl jasmonate and propyl jasmonate.

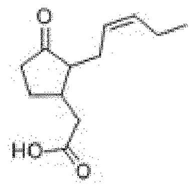

(A)

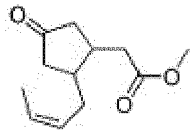

(B)

A "tissue repair agent" is to be understood as a compound or mixture of compounds (in this invention a plant cell culture or a plant cell culture lysate) which due to its inherent capability of recovering damaged cells, or interstitial structures, namely skin or mucosa cells, promote the recovering of the entire functionality of the tissue. An example of tissue repair agent is a compound or mixture of compounds that allows the synthesis of collagen.

The term "a compound derived from hyaluronic acid" or "hyaluran" or "hyaluronic acid compound" (interchangeable used herein) means the anionic, nonsulfated glycosaminoglycan, which can be in the acid form, as well as in the form of an organic or inorganic salt. Hyaluronan is a common ingredient in skin-care products. The presence of hyaluronic acid in epithelial tissue has been shown to promote keratinocyte proliferation and increase the presence of retinoic acid, causing skin hydration. These benefits make hyaluronic acid a very effective topical humectant.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "effective amount" as used herein, means an amount of an active agent high enough to deliver the desired benefit (either the treatment or prevention of the illness), but low enough to avoid serious side effects within the scope of medical judgment.

The term "cosmetically acceptable" or "dermatological acceptable", which is herein used interchangeably, refers to the excipients or carriers suitable for use in contact with human skin without undue toxicity, incompatibility, instability, allergic response, among others.

The term "hydrating agent" or "moisturizer" or "moisturizing agent" which is herein used interchangeably refers to a material which increases the water content of the skin and helps keep it soft and smooth.

The term "emollient" agent refers to a material that softens and soothes the skin in order to correct dryness and scaling of the skin, lubricating the skin surface, encouraging skin water retention, and altering product textures. Non limiting examples of emollients include mallow plant derivatives (extracts) and Chamomile plant derivatives (extracts).

The term "solvent" includes any liquid of organic or inorganic nature that can dissolve a solute to obtain a solution. Examples of inorganic solvents include water. Organic solvents include lipophilic solvents and hydrophilic solvents. The term "hydrophilic" solvent refers to solvents that are capable of creating hydrogen bonding, enabling them to be dissolved more readily in water, and in other polar solvents. The term "lipophilic" solvent refers to non-polar solvents that have little or no capacity to form hydrogen bonds, enabling them to be dissolved in fats, oils, lipids, and other non-polar solvents. Examples of hydrophilic solvents refer to glycols, such as ethylene glycol, propylene glycol, butylene glycol, diethylene glycol, tetramethylene glycol, or 1,2,3-propanetriol (glycerin). Examples of lipophilic solvents include triglyceride derivatides, vegetable oils, mineral oils, animal fats, their fractions and mixtures.

The term "viscosity agent" which is herein used interchangeably refers to a material that increases its viscosity without substantially modifying its other properties.

The term "antioxidant" refers to a material that slows or prevents the oxidation of other molecules. Antioxidants include free radical scavengers and reducing agents. Non limiting examples of antioxidants are ascorbic acid and the salts teherof, and ethylenediaminetetraecetic acid (EDTA).

The term "pH-regulating agent" or "pH buffer" (herewith interchangeably used) refers to acids or bases that can be used to adjust the pH of the finished product to the desired level, without affecting the stability of the solution.

The term "gelling agent" refers to some thickening agents forming a gel, dissolving in the liquid phase as a

colloid mixture that forms a weakly cohesive internal structure.

The term "preservative" refers to a material that prevents or reduces or slows down microbial growth, providing that the stability of the solution is not affected. Non-limiting examples of preservatives include parabens, such as methylparaben, propylparaben, and urea derivatives such as imidazolidinyl urea.

The term "medical device" means as defined by the European Directive 2007/47/EC any product, instrument, device, apparatus, computer program, material or article used in the medico sanitary field and being regulated by the European Directive 90/385/CEE of active implantable medical device, 98/79/CE of medical devices for in vitro diagnostic, and 93/42/CEE for general medical devices. The medical device are included in the sanitary technologies. They are used in human beings for the diagnostic, prevention, control, treatment or alleviation of a disease, for the compensation of a deficiency; for investigating, substituting or modification of anatomy or a physiologic process; and for the regulation of contraception. Medical devices of the present invention relate to compositions or formulations ("medical device compositions"), or to products or devices comprising said compositions or formulations, comprising an effective amount of a plant cell culture from a plant of the *Centella* genus, said plant cell culture comprising an amount of madecassoside comprised from 75 % to 95 % by weight in respect of the total weight of centellosides in the cell culture. The term "medical device composition" is used herein to identify the compositions according to the invention, which compositions, as such (by their selves) are catalogued by the competent sanitary authorities as medical devices.

For the purposes of the invention, any ranges given include both the lower and the upper end-points of the range.

In a preferred embodiment of the first aspect of the invention, the composition is a pharmaceutical composition comprising, together with any acceptable pharmaceutically excipients and/or carriers, an effective amount of a plant cell culture from a plant of the *Centella* genus, said plant cell culture comprising an amount of madecassoside comprised from 75 % to 95 % by weight in respect of the total weight of centellosides in the cell culture.

The repair of the damaged tissues is the result, as will be illustrated in the examples below, of the plant cell culture from a plant of the *Centella* genus with the specific amount of madecassoside. In addition, other of the components of the composition act together with the plant cell culture, and aid to promote also this effect.

In another preferred embodiment, the composition is a cosmetic composition comprising, together with any acceptable cosmetically excipients and/or carriers, an effective amount of a plant cell culture from a plant of the *Centella* genus, said plant cell culture comprising an amount of madecassoside comprised from 75 % to 95 % by weight in respect of the total weight of centellosides in the cell culture.

The cosmetic effect as skin care agent is also due to the plant cell culture from a plant of the *Centella* genus and can be improved with other ingredients, such as phytosterols, flavonoids, natural cell antioxidants, etc.

Yet in another preferred embodiment of the composition according to the invention, the plant cell culture from a plant of the *Centella* genus is comprised in an amount from 1.0 % to 10.0 % by weight in respect of the total weight of the composition. In a preferred embodiment the plant cell culture is comprised from 1.0 % to 5.0 % by weight in respect of the total weight of the composition. In another preferred embodiment the plant cell culture is comprised from 5.0 % to 10.0 % by weight in respect of the total composition. Preferred percentages are 1.0 %, 2.0 %, 3.0 %, 4.0 %, 5,0 %, 6.0 %, 7.0 %, 8.0%, 9.0%, and 10.0%.

In another embodiment, the composition according to the invention, further comprises a compound derived from hyaluronic acid, which is selected from the group consisting of hyaluronic acid, a salt of hyaluronic acid, and mixtures thereof. Hyaluronic acid or derivatives thereof may also act in the composition as an humectant agents due to its hygroscopic properties.

Salts of hyaluronic acid include inorganic salts like salts of alkaline or alkaline earth metals. Examples of alkaline salts of hyaluronic acid include the sodium salt and the potassium salt. Examples of alkaline earth metals refer to calcium salt and magnesium salt.

In a preferred embodiment, the compound derived from hyaluronic acid is comprised in an amount from 0.05 % to 0.50 % by weight of the total composition. Preferred amounts include 0.05 %, 0.10 %, 0,15 %, 0.20 %, 0,25 %, 0.30 %, 0.35 %, 0.40 %, 0.45 % and 0.50 % by weight of the total composition.

In another embodiment of the first aspect of the invention, the plant of the *Centella* genus is *Centella asiatica.* The plant cell culture comes preferably from an undifferentiated cell, although it can be a cell from the leaves, the fruit, the shoots, the buds, the roots, the stems, the branches, the seeds, and the flowers of the plant of *Centella* genus. *Centella asiatica* can be of different territorial origin. Examples of said origins include Asiatic countries, such as China, India, Indonesia, Pakistan, Sri Lanka, African countries, such as Madagascar, oriental European countries, and American countries, such as Mexico.

For the obtention of the plant cell culture, the use of the plant cell cultures technology is specially preferred, and especially the plant cell cultures being the result of suspension cultures, especially of undifferentitaed cell suspension cultures. This technology allows obtaining the products of interest, in this case a plant cell culture enriched in madecassoside, in a highly reproducible way. With this technology the manufacture batches are as similar as possible, and this means a great advantage, because reproducibility is an obliged condition for agents to be approved by regulatory authorities and to be used in pharmaceutical compositions.

Also in a preferred embodiment, the plant cell culture from a plant of *Centella* genus as ingredient in the composition comprises the compound madecassoside in an amount comprised from 85 % to 95 % by weight in respect of the total weight of centellosides in the cell culture. Most preferred amounts are 86 %, 87 %, 88 %, 89 %, 90 %, 91 %, 92 %, 93 %, 94 %, and 95 % by weight in respect of the total weight of centellosides in the cell culture. Specially preferred is an amount of 90 % by weight of madecassoside in respect of the total weight of centellosides.

In another preferred embodiment, the plant cell culture comprises an amount of madecassoside from 85 % to 95 % by weight in respect of the total weight of centellosides in the plant cell culture; and an amount of asiaticoside which goes from 5 % to 15 % by weight in respect of the total weight of centellosides in the plant cell culture, thus representing both 100 % by weight of the centellosides of the cell culture. Most preferably, the plant cell culture of *Centella* genus comprises an amount of 90 % by weight of madecassoside in respect of the total weight of centellosides, and an amount of 10 % by weight of asiaticoside in respect of the total weight of centellosides.

In another embodiment, both madecassoside and asiaticoside represent the majoritary of the centellosides in the cell culture, both being at least the 97 %, at least the 98%, at least the 99 %, or at least 99.9 % by weight of the total weight of centellosides in the cell culture, and wherein the proportion of madecassoside is a least 75 % by weight of the total weight of centellosides in the cell culture, preferably 85 % and most preferably 90 %. Other centellosides which can be present are asiatic acid and madecassic acid, which in the cell culture are generally present in minor amounts.

An example of a method for obtaining the plant cell culture to be used as ingredient in the pharmaceutical, cosmetical and/or medical device composition of the invention comprises the steps of:

a) cultivating a plant cell from a plant of the *Centella* genus in a suitable medium for a period comprised from 8 to 15 days, until stationary growing phase is reached, in order to obtain an in-stationary phase culture;
b) adding from 2 to 6 dosis of a jasmonate compound to the growing culture for a period of time comprised from 13 to 18 days, wherein each one of the dosis added corresponds to an amount such of jasmonate compound that the final concentration at the end of each addition is from 80 μM to 150 μM.

As above indicated the plant cell culture is preferably obtainable by cultivating by cell suspension an undifferentiated plant cell from a plant of the *Centella* genus in a suitable medium. The examples below show a method of obtention of a plant cell culture by suspension of undifferentiated cells, in which 3 dosis of a jasmonate compound (methyl jasmonate) are added to the growing culture for a period of time comprised from 13 to 18 days. Each one of the dosis added corresponds to an amount such of jasmonate compound that the final concentration at the end of each addition is from 80 μM to 150 μM.

The plant cells of the *Centella* genus can be added in the composition as entire cells, as well as in the form of lysate cell culture. Thus, another embodiment relates to the composition wherein the plant cell culture is in form of a plant cell culture lysate.

The plant cell culture lysate is preferably obtained by mechanical disruption of a cell suspension culture of the plant cell culture, and further filtration through a mesh from 50 to 150 μm of pore diameter. Other means for lysing the plant cell cultures are also applicable provided that the final mixture is proper for being administered or used in derivative products, such as pharmaceutical or cosmetical compositions. Examples of such means include efreezing at very low temperature. With the lysis the mixture of the plant cell culture comprises compounds of the culture medium not consumed by the cells; compounds secreted by the cells to the medium; soluble intracellular compounds; and not soluble cell membrane compounds.

In another preferred embodiment, the composition further comprises an extract of a plant of the genus selected from the group consisting of camomille, mallow, red clover, and soja. Preferred extracts include *Malva sylvestris* extract, and *Matricaria Chamomilla* extract.

The compositions according to the invention, which are pharmaceutical or cosmetic compositions, or the medical devices (sanitary products) comprising the compositions, comprise pharmaceutically or cosmetically excipients and/or carriers selected from the group consisting of a solvent or vehicle, a viscosity agent, an hydrating agent, a gelling agent, an emollient, a pH-regulating agent, an antioxidant, a preservative agent, and a mixture thereof.

Examples of vehicles include, but are not limited to, water, propylene glycol, butylene glycol, ethanol, isopropanol, or silicones. Preferably, the vehicle is water. In another preferred embodiment the vehicle is a mixture of water and propylene glycol, wherein the amount of propylene glycol in the final composition is 10 % by weight. As commonly known the amount of the vehicle is always adjusted or added in order that the amounts of the ingredients of the composition yield 100 %, which is that the vehicle is added in "as needed for" (q.s.p) amount.

Examples of appropriate viscosity agents include, but are not limited to, cellulose or their derivatives such as acrylates, hydroxypropyl methylcellulose,
polyethylene glycol, microcrystalline cellulose, cetearyl alcohol, alginates, branched polysaccharides, fumed silica, xanthan gum, carbomer, and polyacrylates. Preferably, the viscosity agent is selected group consisting of microcrystalline cellulose, cetearyl alcohol, cellulose, xanthan gum, and carbomer. Preferably, the viscosity agent is an acrylate. The

amount of the viscosity agents in the compositions of the present invention is comprised from 0.5 % to 2% by weight.

Examples of emollients include, but are not limited to, octyl hydroxystearate, lanolin, caprylic/capric triglyceride, cetyl palmitate, octyldodecanol, cetyl alcohol, isopropyl isostearate, glyceryl dilaurate, isopropyl myristate, palm alcohol, dimethicone, squalane, plukenetia volubilis seed oil, butyrospermum parkii butter, sucrose cocoate, plant extracts or their mixtures. Preferably the emollient is a plant extract selected from the group consisting of mallow plant derivatives (extracts), Soy plant derivatives (extracts), Red Clover plant derivatives (extracts), and Chamomile plant derivatives (extracts) or their mixtures. Most preferred examples of plant extracts include, as above indicated, *Malva sylvestris* extract, and *Matricaria Chamomilla* extract or their mixtures. The amount of the emollients in the compositions of the present invention is comprised from 1.0 % to 2.0 % by weight.

Examples of appropriate pH-regulating agents include, but are not limited to, acetic acid, lactic acid, citric acid, ethanolamine, formic acid, oxalic acid, potassium hydroxide, sodium hydroxide, triethanolamine, or their mixtures. Preferably, the pH-regulating agent is triethanolamine. The amount of the pH-regulating agents in the compositions of the present invention is comprised from 0.2 % to 0.9% by weight.

Examples of appropriate antioxidants include, but are not limited to, free radical scavengers or reducing agents such as, ascorbic acid, ethylenediaminetetraecetic acid (EDTA) and the salts thereof, acetyl cysteine, ascorbyl palmitate, butylated hydroxytoluene, green tea extract, caffeic acid, cysteine, tocopherol, ubiquinone, propyl gallate, butylated hydroxytoluene (BHT), and their mixtures. Preferably, the antioxidant is selected from ascorbic acid, ethylenediaminetetraacetic acid (EDTA) and their mixtures. The amount of the antioxidant agents in the compositions of the present invention is comprised from 0.001 % to 0.1% by weight.

Examples of appropriate preservative agents include, but are not limited to, benzoic acid, butylparaben, ethylparaben, propylparaben, methylparaben, sorbic acid, potassium sorbate, sodium benzoate, phenoxyethanol, triclosan, and urea derivatives or their mixtures. Preferably, the preservative agent is selected group consisting of methylparaben, propylparaben, and imidazolidinyl urea. The amount of the preservative agents in the compositions of the present invention is comprised from 0.02 % to 0.32 % by weight.

When the composition is a pharmaceutical composition as defined above, it is preferably for use as a medicament.

Thus, in a preferred embodiment, the present invention refers to the pharmaceutical composition as defined above, for use in the prophylaxis and/or treatment of a disease or condition which occurs with tissue damage, in particular topical skin (epidermis) or mucosa tissue damage. Thus, the composition of the invention is also for use as a medicament.

When the composition according to the invention is used as a medicament, it is preferably for use as tissue repair agent. This aspect can also be formulated as a method for treating and/or preventing tissue damage, namely topical skin (epidermis) or mucosa tissue damage, the method comprising administering a composition comprising a plant cell culture of the *Centella* genus, said plant cell culture comprising from 75 % to 95 % by weight of madecassoside, in respect of the total weight of centellosides in the cell culture, to the subject in need thereof. This aspect can alternatively be formulated as the use of a composition comprising a plant cell culture of the *Centella* genus, said plant cell culture comprising from 75 % to 95 % by weight of madecassoside in respect of the total weight of centellosides in the cell culture for the manufacture of a medicament for the treatment and/ or prevention of tissue damage, namely topical skin (epidermis) or mucosa tissue damage. The plant cell culture of the *Centella* genus itself may be also for use in the treatment or prevention of tissue damage, said plant cell culture comprising from 75 % to 95 % by weight of madecassoside in respect of the total weight of centellosides in the cell culture.

The examples below clearly demonstrate that with the pharmaceutical composition of the invention the synthesis of collagen is induced when administered in a model of skin cells, namely in human fibroblasts.

In a preferred embodiment the composition according to the invention is in the form of a topical composition.

Thus, in an embodiment, the composition according to the invention is a topical pharmaceutical composition, and comprises an effective amount of a plant cell culture from a plant of the *Centella* genus, said plant cell culture comprising an amount of madecassoside comprised from 75 % to 95 % by weight in respect of the total weight of centellosides in the cell culture, together with one or more appropriate topical pharmaceutically acceptable excipients and/or carriers. Thus, the pharmaceutical composition is a topically administrable composition, which can be administered to the skin (epidermis) or mucous membranes (the vagina, anus, throat, eyes and ears). Additionally, the compositions of the present invention may contain other ingredients, such as fragrances, colorants, and other components known in the state of the art for use in topical formulations.

The topical compositions of the invention can be formulated in several forms that include, but are not limited to, solutions, aerosols and non-aerosol sprays, shaving creams, powders, mousses, lotions, gels, sticks, ointments, pastes, creams, shampoos, shower gel, body washes or face washes. A preferred formulation is a gel. Thus, in another preferred embodiment the compositions are in the form of a gel.

A "gel" is a solid, jelly-like material that can have properties ranging from soft and weak to hard and tough. Gels are defined as a substantially dilute cross-linked system, which exhibits no flow when in the steady-state. By weight, gels are mostly liquid, yet they behave like solids due to a three-dimensional cross-linked network within the liquid. It is

the crosslinks within the fluid that give a gel its structure (hardness) and contribute to stickiness (tack). In this way gels are a dispersion of molecules of a liquid within a solid in which the solid is the continuous phase and the liquid is the discontinuous phase.

Topical compositions of the present invention can be prepared according to methods well known in the state of the art. The appropriate excipients and/or carriers, and their amounts, can readily be determined by those skilled in the art according to the type of formulation being prepared.

Although topical administration is preferred, other administration routes are possible, such as oral administration. Therefore, the composition containing the effective amount of the plant cell culture from *Centella* genus of the invention can be administered orally in form of tablets, pills, capsules, microcapsules, granules, suspensions, syrups, freeze-dried powders, liquid preparations, etc. Selection of the excipients and the most appropriate methods for formulation in view of the particular purpose of the composition (topical, injectable or oral administration) is within the scope of ordinary persons skilled in the art of pharmaceutical technology.

The cosmetic composition according to the invention is used as a skin care agent, wherein the skin care comprises ameliorating at least one of the following symptoms comprises ameliorating at least one of the following symptoms: roughness, dehydration, tightness, and lack of elasticity.

Thus, in an embodiment, the composition according to the invention is a cosmetic composition, which is also in the form of a topical composition, and preferably in the form of a gel. In another embodiments, the cosmetic composition can be formulated in several other forms that include, but are not limited to, solutions, aerosols and non-aerosol sprays, shaving creams, powders, mousses, lotions, sticks, ointments, pastes, creams, shampoos, shower gel, body washes or face washes.

The pharmaceutical, cosmetic and/or medical device compositions according to the invention may be declared themselves as a medical device. For example, the gel formulation itself can be used as medical device, that is, catalogued as a medical device. In the alternative, the medical device in the sense of the invention include any other device, which apart of other components, comprises a pharmaceutical cosmetic and /or medical device composition as defined above. That is, a pharmaceutical, cosmetic, and/or medical device composition comprising in turn, together with any acceptable pharmaceutical or cosmetically excipient and/or carrier, an effective amount of a plant cell culture from a plant of the *Centella* genus, said plant cell culture comprising an amount of madecassoside comprised from 75 % to 95 % by weight in respect of the total weight of centellosides in the cell culture.

Examples of these medical devices, which include other components and the composition according to the invention, relate to woven or non-woven fabrics, in which a layer or coating of the composition comprising the effective amount of a plant cell culture from a plant of the *Centella* genus, is totally or partially distributed onto the surface of the fabric, thus obtaining fabrics with the properties conferred by the composition according to the invention.

The medical device of the invention can be used as a medicament. When used as a medicament, it is preferably for tissue repairing.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

EXAMPLES

Example 1: Obtention of plant cell cultures of *Centella asiatica.*

Calli were obtained using Murashige and Skoog (MS) medium supplemented with 25-40 g $l^{-1}$ of sucrose (for example, 35 g $l^{-1}$), between 2-4 mg $l^{-1}$ of 6-benzyladenine (BA) (for example 3.3 mg $l^{-1}$) and between 2-4 mg $l^{-1}$ of 1-naphthaleneacetic acid (NAA) (for example, 2.5 mg $l^{-1}$), which provided good callus induction. As a growth medium, MS medium was used supplemented with 25-40 g $l^{-1}$ of sucrose (for example 40 g $l^{-1}$), between 1.5-4 mg $l^{-1}$ of 2,4-dichlorophenoxyacetic acid (2,4-D) (for example, 2.5 mg $l^{-1}$) and between 2.5-4 mg $l^{-1}$ of N1-(2-chloro-4-pyridyl)-N2 phenylurea (4PU-30), for example (2.5 mg $l^{-1}$) to obtain large and friable calli.

To establish cell suspension cultures, callus pieces (20 g) were inoculated into 500-ml flasks containing 200 ml of MS liquid medium supplemented with 20-30 g of sucrose (for example, 20 g), between 1-3 mg $l^{-1}$ of 2,4-D (for example, 1 mg $l^{-1}$) and between 0.1-1 g $l^{-1}$ of BA (for example, 0.8 g $l^{-1}$) and placed in a rotary shaker at 100 rpm in the dark at 25 °C. Cell cultures were passed through a 60-μm nylon filter every 2 weeks for 6 months to obtain a fine cell suspension culture.

For the cell suspension assays, 20 g of cells (fresh weight, FW) were inoculated into 200 ml of the aforementioned liquid medium in 500-ml flasks, with or without the elicitor, and were maintained in a rotary shaker at 100 rpm in the dark

at 25 °C. After 12 days of growing (when stationary growing phase is reached), the cell suspension was elicitated with 100 μM methyl jasmonate (MeJA) at days 12, 16 and 22 from the initiation of the suspension. Other possible elicitation days include, a part of the day in which until stationary growing phase is reached and the elicitation schedule is initiated, from 3 to 5 days later of the first elicitation; and from 8 to 11 later of the first elicitation.

Afterwards, the elicited cultured was lysed with an homogenizer at 10000-15000 rpm, and filtered through a nylon mesh with a diameter pore of 100 μm.

The mixture contained:

- compounds of the culture medium not consumed by the cells;
- compounds secreted by the cells to the medium;
- soluble intracellular compounds; and
- not soluble cell membrane compounds with a size lower than 100 μm.

The final product was a cell culture lysate with high contents of madecassoside in respect of the total centellosides in the C. *asiatica* cells.

HPLC analysis of the cell culture lysate indicated that the amount of madecassoside was 90 % by weight of the total weight of centellosides (asiaticoside, asiatic acid, and madecassic acid representing 10 % by weight of the total weight of centellosides).

The amounts of centellosides were determined as indicated in Example 2 below. The areas under the curves (or peaks, a.u.c) of each centelloside of the HPLC analysis were calculated and correlated with the amount of each compound. Finally, the percentage by weight of each centelloside (madecassoside, asiaticoside, madecassic acid, and asiatic acid) in relation to the total weight of centellosides in the lysate were determined as:

$$\% = [(\text{ a.u.c of a specific centelloside}) / (\sum \text{ a.u.c of all centellosides})] \times 100$$

In FIG. 2, it is shown the amount (mg/L) of madecassoside (M) and of asiaticoside (A) obtained in the cell suspension culture with methyl jasmonate (100 μM). Left bars of the figure show the amounts determined with one (1 X) addition (or treatment; (T)) of methyl jasmonate. Right bars correspond to the amounts of madecassoside (M) and asiaticoside (A) obtained with the elicitation schedule disclosed above; that is, three additions (3X) at days 12, 16 and 22 from the initiation of the suspension, and determined at day 27 from the initiation of the suspension. Data depicted in FIG. 2 correspond to the total concentrations from the cell fraction and the culture liquid medium fraction.

As can be seen, with the three additions of methyl jasmonate the absolute amount of madecassoside was from 8 to 9 times higher than the amount of asiaticoside, namely 8,5 times higher.

As deducible from this example, the jasmonate compound is added three times with the following general schedule:

(i) the first dosis once the growing culture has reached the stationary growing phase;
(ii) the second dosis is added from 3 to 5 days later in respect of the first addition;
(iii) the third dosis is added from 8 to 11 days later in respect of the first addition. The first dosis is generally the dosis which starts step b) of the method of obtaining the plant cell culture of the plant of *Centella* genus. The step in which the stationary phase is reached, or of growing, is generally controlled or followed by means of turbidimetric assays and packing volum determination.

Example 2. Determination of centellosides (HPLC).

The quantification of the four centellosides (asiaticoside, madecassoside, asiatic acid and madecassic acid) was carried out through high-performance liquid chromatography (HPLC).

For cells extraction,1 g of fine powder (lyophilised) was first sonicated in methanol:water (9:1, v/v; 2 x 25 mL) for 2 h at room temperature and filtered through a 0.45 μm filter (Waters Millipore). The filtrate was evaporated to dryness. The dried extract was disolved in 1 mL of methanol and filtered through a 0.45 μm filter (Waters Millipore) and the clear filtrate was used for HPLC analysis. HPLC-UV analysis was performed at room temperature with a Spherisorb 5μm ODS2 4.6x250 mm column (Waters, Milford, MA, USA) using gradient elution, the eluents being acetonitrile (A) and water with ammonium dihydrogen phosphate 10 mM (pH 2.5 with orthophosphoric acid) (B) according to the following profile: 0-15 min, 80% A; 15-30 min, 62% A; 30-37 min, 30% A; 37-40 min, 80% A. The flow rate was 1 ml min-1 and the detector was set at 214 nm.

Example 3. Comparative assay in an *in vitro* model

In order to illustrate the advantageous effects of the plant cell culture of the invention, following it is disclosed an assay with different compositions comprising a cell culture lysate of *Centella asiatica* according to the invention. The capability of the compositions of inducing collagen synthesis of this cell culture lysate is tested in a culture of human dermal fibroblasts.

Table 1 show the characteristics of the tested compositions.

Table 1. Tested products

| Raw material | Concentration (% by weight) | | | |
|---|---|---|---|---|
| | PR/52/A | PR/53/A | PR/54/A | PR/55/A |
| *Centella asiatica* cell culture lysate | 10,000 | 5,000 | 1,000 | 0 |
| *Centella asiatica* extract with a 90 % of madecassoside in respect of all centellosides (from Guangxi Changzhou Natural products Development (China); powder extract of the dried whole plant) | 0 | 0 | 0 | 0,500 |
| Excipients (solvents, emollients, pH buffers, gelling agents, antioxidants and preservative agents) | qsp. 100 * | | | |
| * "q.s.p" means "as needed for". | | | | |

The aim of this assay was to see the effect of the formulations listed in Table 1. Three of them (PR/52/A; PR/53/A; and PR/54/A) contained a *Centella asiatica* cell culture lysate, prepared as exposed in Example 1. They contained an amount of madecassoside comprised from 75 % to 95 %, namely 90 % by weight in respect of the total weight of centellosides obtained after the treatment with the elicitor methyl jasmonate. The other formulation (PR/55/A) contained an amount of a *Centella asiatica* extract.

The test consisted in the evaluation of the effect in the collagen synthesis in human dermis fibroblasts after 24 hours of treatment with these formulations, each one applied at different non-toxic concentrations (100, 250 and 500 μg/ml) previously determined.

Collagen was determined with a colorimetric assay using the coloring agent Sirius Red (Direct red 80), which binds specifically to a triple helix sequence of the native collagen. As positive control (Control +) the beta transforming growing factor (TGF-β) was used. As negative control (Control -) culture medium was added.

Cells were cultivated at 37 °C for 24 hours (50 x$10^3$ cel/$cm^2$). The tested formulation was prepared and added to the cells at the three above indicated concentrations and incubated at 37 °C for 24 additional hours. For every tested concentration the following determinations were performed: a) collagen quantification, and b) cell quantification (Trypsin: 0,2 % w/v).

The results of the assay are depicted in FIG. 1, wherein the amount of collagen in terms of μg/$10^6$ human fibroblast cells is indicated in form of bars for every concentration (100, 250, and 500 μg/ml) tested for each formulation. When three bars are showed for a determined formulation, left bar shows the result at a final concentration in the culture of 100 μg/ml; the bar in the middle shows the results at 250 μg/ml; and right bar shows the results at 500 μg/ml. Vertical lines show the standard deviation of the results.

Cell growth was similar in negative control and in the cultures treated with the formulations, thus confirming that the assayed concentrations (or selected doses) of 100, 250, and 500 μg/ml were non-cytotoxic.

As can be deduced from this FIG. 1, the formulation containing 10 % by weight of the *Centella asiatica* cell culture lysate of the invention (PR/52/A) was able to stimulate collagen secretion in the culture medium from 100 to 500 μg/ml, being maximal at 250 μg/ml. This means 42 % more in respect of the negative control. The stimulation represented in turn the 80 % of the positive control stimulation (TGF-β).

On the other hand, the formulation containing 5 % by weight of the *Centella asiatica* cell culture lysate of the invention (PR/53/A) stimulated the collagen secretion (synthesis) between 100 and 500 μg/ml, also being maximal at 250 μg/ml. This represented 65 % more in respect of the negative control. The stimulation was noteworthy the 127 % in relation of the induction by the positive control.

The formulation containing 1 % by weight of the *Centella asiatica* cell culture lysate of the invention (PR/54/A) stimulated the collagen secretion (synthesis) between 100 and 250 μg/ml, also being maximal at 250 μg/ml. The amount of secreted collagen represented an increase of 31 % in respect of the negative control.

Finally, the formulation comprising 0,5 % of an extract of *Centella asiatica* with 90 % of madecassoside

(PR/55/A), that is, a powder of an extract of the dried whole plant, purified and titrated (TECA), stimulated the collagen secretion (synthesis) between 100 and 500 μg/ml, being maximal at 250 μg/ml. The amount of secreted collagen represented an increase of 55 % in respect of the negative control, and the increase was comparable with that of the positive control.

All these data allow concluding that the four formulations were able to stimulate the collagen synthesis, since the amount of collagen obtained in the cultured fibroblasts with all the concentrations of the four tested formulations obtained was greater than the amount in the negative control. Interestingly, the formulation comprising 5 % by weight of the *Centella asiatica* cell culture lysate of the invention (PR/53/A) had a similar effect, even greater, than the positive control. In addition, this formulation (5 % by weight of the *Centella asiatica* cell culture lysate of the invention) had an effect greater than the formulation comprising the extract of the plant.

Example 4. Gel composition for use as tissue repairing formulation.

Next Table 2.1 shows an example of topical pharmaceutical composition comprising as one of the ingredients a lysate from a plant cell culture of *Centella asiatica* according to the invention.

Besides, Table 2.2 shows the ranges of percentage by weight (%) of the components of a composition according to the invention.

Table 2.1 Gel comprising *C. asiatica.*

| Ingredient | Percentage by weight (%) |
|---|---|
| WATER | 80.96 |
| Lysate from a plant cell culture of *Centella asiatica* | 5.000 |
| Acrylates/C10-30 Alkyl acrylate cross-polymer | 0.550 |
| TRIETHANOLAMINE | 0.700 |
| IMIDAZOLIDINYL UREA | 0.200 |
| DISODIUM EDTATE | 0.100 |
| ASCORBIC ACID | 0.030 |
| SODIUM HYALURONATE | 0.100 |
| PROPILENE GLYCOL | 10.000 |
| PROPYLPARABEN | 0.040 |
| METHYLPARABEN | 0.320 |
| CHAMOMILE EXTRACT (*chamomilla recutita* extract) | 1.000 |
| MALLOW EXTRACT (*malva sylvestris* extract) | 1.000 |

Table 2.2. Ranges of the ingredients forming compositions in form of gel according to the invention comprising *C. asiatica.*

| Ingredient | Percentage by weight (%) |
|---|---|
| WATER | q.s.p** 100 |
| Lysate from a plant cell culture of *Centella asiatica* | 1 - 10% |
| Acrylates/C10-30 Alkyl Acrylate cross-polimer | 0.5 - 2.0 % |
| TRIETHANOLAMINE | 0.2 - 0.9% |
| IMIDAZOLIDINYL UREA | 0.03 -0.5% |
| DISODIUM EDTATE | 0.005 - 0.1% |
| ASCORBIC ACID | 0.001 - 0.1% |
| SODIUM HYALURONATE | 0.05-1% |
| PROPILENGLICOL | 10% |
| PROPILPARABEN | 0.02 - 0.1 % |

(continued)

| Ingredient | Percentage by weight (%) |
|---|---|
| METILPARABEN | 0.1 - 0.32% |
| CAMOMILLE EXTRACT (chamomilla recutita extract) | 1.0% |
| MALLOW EXTRACT (malva sylvestris extract) | 1.0% |
| ** "q.s.p" means "as needed for". | |

This gel compositions are indicated in several circumstances in which vulvar and/or vaginal mucosas are altered particularly due to vaginal dryness.

The compositions are able to promote regeneration, flexibility and elasticity of vaginal mucosa during peri-menopause or menopause, in the last days of menstrual cycle, in case of dryness occasioned by some oral contraceptive products, during post-delivery, and in radiotherapy-associated dryness.

In addition, the compositions of the invention may be applied at any skin surface or mucosa, and they are able to speed the healing process in case of surgery, for example in case of an episiotomy. The composition is also useful to reduce wrinkles and scars, as well as to promote healing in case of burnt tissue.

Thus, it has been duly proved that the pharmaceutical composition according to the invention is for use as a medicament, in particular as a medicament for tissue repairing, due to the capability of the composition of inducing collagen synthesis. In other words, the pharmaceutical composition is useful in those situations in which a tissue (dermis, epidermis, internal tissue) has been damaged for example due to an accident or by surgery. The pharmaceutical composition of the invention promotes the healing of the wounds and is thus therapeutically effective.

Besides, the composition is also cosmetically applicable as a skin care agent, since the induction of the collagen synthesis leads to the skin care by ameliorating at least one of the following symptoms: roughness, dehydration, tightness, and lack of elasticity, all of them being processes in which new collagen fibers are needed.

The pharmaceutical, cosmetic, and/or medical device composition of the examples may be applied in a woven or non-woven tissue as a layer or coating to be further contacted with the desired area to be cared. Thus, it can be supplied as a layer in a napkin, a sanitary towel, a basin, a gauze or a band.

REFERENCES CITED IN THE APPLICATION

- Maquart et al., "Stimulation of collagen synthesis in fibroblast cultures by a triterpene extracted from Centella asiatica". Conn. Tissue Res - 1990, vol. 24, pp. 107-120.
- EP867447.
- Mulabagal et al., "Plant cell cultures-an alternative and efficient source for the production of biologically important secondary metabolites", International Journal of Applied Science, Engineering and Technology-2004, Vol. 2, pp.: 101-153.
- Bonfill et al., "Production of centellosides and phytosterols in cell suspension cultures of Centella asiatica", Plant Cell Tiss Organ Cult - 2011, Vol . No. 104, pp.: 61-67.


## Claims

**1.** A pharmaceutical or cosmetic topical composition comprising, together with any acceptable pharmaceutically or cosmetically excipients and/or carriers:

- a compound derived from hyaluronic acid which is selected from the group consisting of hyaluronic acid, a salt of hyaluronic acid, and mixtures thereof; and
- an effective amount of a plant cell culture from *Centella asiatica,* said plant cell culture comprising an amount of madecassoside from 85 % to 95 % by weight in respect of the total weight of centellosides in the plant cell culture; and an amount of asiaticoside from 5 % to 15 % by weight in respect of the total weight of centellosides in the plant cell culture; and

wherein the plant cell culture is obtainable by a method comprising:

a) cultivating an undifferentiated plant cell from *Centella* asiatica by cell suspension in a suitable medium for a period comprised from 8 to 15 days, until stationary growing phase is reached, in order to obtain an in-stationary phase culture;
b) adding from 2 to 6 dosis of a jasmonate compound to the growing culture for a period of time comprised from 13 to 18 days, wherein each one of the doses added corresponds to an amount such of jasmonate compound that the final concentration at the end of each addition is from 80 $\mu$M to 150 $\mu$M.

**2.** The pharmaceutical or cosmetic composition according to claim 1, wherein in step (b) the jasmonate ocmpound is added three times with the following schedule;

(i) the first dose once the culture of step a) has reached the stationary growing phase;
(ii) the second dose is added from 3 to 5 days later in respect of the first addition;
(iii) the third dose is added from 8 to 11 days later in respect of the first addition.

**3.** The composition according to any one of the claims 1-2, wherein the plant cell culture from *Centella asiatica* is comprised in an amount from 1.0 % to 10.0 % by weight of the total composition.

**4.** The composition according to any of claims 1-3, wherein the compound derived from hyaluronic acid is comprised in an amount from 0.05 % to 0.5 % by weight of the total composition.

**5.** The composition according to any one of claims 1-4, wherein the plant cell culture is in form of a plant cell culture lysate.

**6.** The composition according to any one of claims 1-5, which further comprises an extract of a plant selected from the group consisting of *Malva sylvestris* extract, and *Matricaria Chamomilla* extract.

**7.** The composition according to any one of claims 1-6, which is a gel.

**8.** A medical device comprising the composition as defined in any of the preceeding claims.

**9.** The pharmaceutical composition as defined in any one of claims 1-7 or the medical device of claim 8 for use as a medicament.

**10.** The pharmaceutical composition or the medical device for the use of claim 9, wherein the medicament is for skin tissue repairing.

**11.** Use of a topical cosmetic composition as defined in any of the claims 1-7 or the medical device of claim 8, as a skin care agent, wherein the skin care comprises ameliorating at least one of the following symptoms: roughness, dehydration, tightness, and lack of elasticity.

**Patentansprüche**

**1.** Eine pharmazeutische oder kosmetische topische Zusammensetzung umfassend, zusammen mit etwaigen pharmazeutisch oder kosmetisch akzeptablen Hilfsstoffen und/oder Trägerstoffen:

- eine Verbindung, die aus Hyaluronsäure abgeleitet ist, welche ausgewählt aus der Gruppe bestehend aus Hyaluronsäure, einem Salz von Hyaluronsäure, und Mischungen davon ist; und
- eine wirksame Menge von einer Pflanzenzellkultur von *Centella asiatica,* wobei die Pflanzenzellkultur eine Menge von Madecassosid von 85 Gew.-% bis 95 Gew.-% bezogen auf das gesamte Gewicht von Centellosiden in der Pflanzenzellkultur umfasst; und eine Menge von Asiaticosid von 5 Gew.-% bis 15 Gew.-% bezogen auf das gesamte Gewicht von Centellosiden in der Pflanzenzellkultur umfasst; und

wobei die Pflanzenzellkultur durch ein Verfahren erhältlich ist, welches folgendes umfasst:

a) das Kultivieren von einer undifferenzierten Pflanzenzelle von *Centella asiatica* durch Zellsuspension in einem geeigneten Medium für einen Zeitraum von 8 bis 15 Tagen, bis die stationäre Wachstumsphase erreicht wird, um eine Kultur in stationärer Phase zu erhalten;
b) das Hinzufügen von 2 bis 6 Dosen von einer Jasmonatverbindung in die wachsende Kultur für einen Zeitraum

von 13 bis 18 Tagen, wobei jede hinzugefügte Dose einer Menge der Jasmonatverbindung entspricht, bei der die Endkonzentration am Ende von jeder Zugabe von 80 μM bis 150 μM reicht.

**2.** Die pharmazeutische oder kosmetische Zusammensetzung nach Anspruch 1, wobei im Schritt (b) die Jasmonatverbindung dreimal mit dem folgenden Zeitplan hinzugefügt wird;

(i) die erste Dose: wenn die Kultur des Schritts a) die stationäre Wachstumsphase erreicht hat;
(ii) die zweite Dose: von 3 bis 5 Tagen nach der ersten Zugabe;
(iii) die dritte Dose: von 8 bis 11 Tagen nach der ersten Zugabe.

**3.** Die Verbindung nach einem der Ansprüche 1-2, wobei die Pflanzenzellkultur von *Centella asiatica* in einer Menge von 1,0 Gew.-% bis 10,0 Gew.-% der gesamten Verbindung enthalten ist.

**4.** Die Verbindung nach einem der Ansprüche 1-3, wobei die aus Hyaluronsäure abgeleitete Verbindung in einer Menge von 0,05 Gew.-% bis 0,5 Gew.-% der gesamten Verbindung enthalten ist.

**5.** Die Zusammensetzung nach einem der Ansprüche 1-4, wobei die Pflanzenzellkultur in Form eines Pflanzenzellkulturlysats ist.

**6.** Die Zusammensetzung nach einem der Ansprüche 1-5, welche weiterhin einen Extrakt von einer Pflanze umfasst, der ausgewählt aus der Gruppe bestehend aus Extrakt von *Malva sylvestris* und Extrakt von *Matricaria Chamomilla* ist.

**7.** Die Zusammensetzung nach einem der Ansprüche 1-6, welche ein Gel ist.

**8.** Eine medizinische Vorrichtung umfassend die Zusammensetzung wie in einem der vorhergehenden Ansprüche definiert.

**9.** Die pharmazeutische Zusammensetzung wie in einem der Ansprüche 1-7 definiert oder die medizinische Vorrichtung des Anspruchs 8 zur Verwendung als Arzneimittel.

**10.** Die pharmazeutische Zusammensetzung oder die medizinische Vorrichtung zur Verwendung nach Anspruch 9, wobei das Arzneimittel zur Hautgewebereparatur ist.

**11.** Verwendung von einer topischen kosmetischen Zusammensetzung wie in einem der Ansprüche 1-7 definiert oder der medizinischen Vorrichtung des Anspruchs 8, als Hautpflegemittel, wobei die Hautpflege die Besserung von mindestens einem der folgenden Symptome umfasst: Rauheit, Austrocknung, Straffheit und fehlende Elastizität.

## Revendications

**1.** Une composition topique pharmaceutique ou cosmétique comprenant, conjointement avec des excipients et/ou véhicules pharmaceutiquement acceptables quelconques :

- un composé dérivé d'acide hyaluronique qui est choisi dans le groupe constitué d'acide hyaluronique, un sel d'acide hyaluronique, et des mélanges de ceux-ci ; et
- une quantité efficace d'une culture cellulaire végétale de *Centella asiatica,* comprenant ladite culture cellulaire végétale une quantité de madécassoside allant de 85 % à 95 % en poids par rapport au poids total de centellosides dans la culture cellulaire végétale ; et une quantité d'asiaticoside allant de 5 % à 15 % en poids par rapport au poids total de centellosides dans la culture cellulaire végétale ; et

dans laquelle la culture cellulaire végétale peut être obtenue par un procédé comprenant :

a) cultiver moyennant suspension cellulaire une cellule végétale indifférenciée de *Centella asiatica* dans un milieu approprié pendant une période allant de 8 à 15 jours, jusqu'à ce que la phase de croissance stationnaire soit atteinte, afin d'obtenir une culture en phase stationnaire ;
b) additionner de 2 à 6 doses d'un composé de jasmonate à la culture en croissance pendant une période de temps allant de 13 à 18 jours, où chacune des doses ajoutées correspond à une quantité du composé de

jasmonate telle que la concentration finale à la fin de chaque addition est de 80 µM à 150 µM.

2. La composition pharmaceutique ou cosmétique selon la revendication 1, dans laquelle dans l'étape (b) le composé de jasmonate est additionné trois fois avec le programme suivant ;

   (i) la première dose lorsque la culture de l'étape a) a atteint la phase de croissance stationnaire ;
   (ii) la deuxième dose est ajoutée de 3 à 5 jours après la première addition ;
   (iii) la troisième dose est ajoutée de 8 à 11 jours après la première addition.

3. La composition selon l'une quelconque des revendications 1-2, dans laquelle la culture cellulaire végétale de *Centella asiatica* est comprise dans une quantité de 1,0 % à 10,0 % en poids de la composition totale.

4. La composition selon l'une quelconque des revendications 1-3, dans laquelle le composé dérivé d'acide hyaluronique est compris dans une quantité de 0,05 % à 0,5 % en poids de la composition totale.

5. La composition selon l'une quelconque des revendications 1-4, dans laquelle la culture cellulaire végétale est sous forme d'un lysat de culture cellulaire végétale.

6. La composition selon l'une quelconque des revendications 1-5, qui comprend en outre un extrait d'une plante choisi dans le groupe constitué d'extrait de *Malva sylvestris* et extrait de *Matricaria Chamomilla.*

7. La composition selon l'une quelconque des revendications 1-6 qui est un gel.

8. Un dispositif médical comprenant la composition telle que définie dans l'une quelconque des revendications précédentes.

9. La composition pharmaceutique telle que définie dans l'une quelconque des revendications 1-7, ou le dispositif médical de la revendication 8 pour son utilisation en tant que médicament.

10. La composition pharmaceutique ou le dispositif médical pour l'utilisation de la revendication 9, où le médicament est destiné pour la réparation du tissu de la peau.

11. Utilisation d'une composition cosmétique topique telle que définie dans l'une quelconque des revendications 1-7, ou du dispositif médical de la revendication 8, comme agent pour le soin de la peau, dans laquelle le soin de la peu comprend améliorer au moins un des symptômes suivants : la rugosité, la déshydratation, la raideur, et la manque d'élasticité.

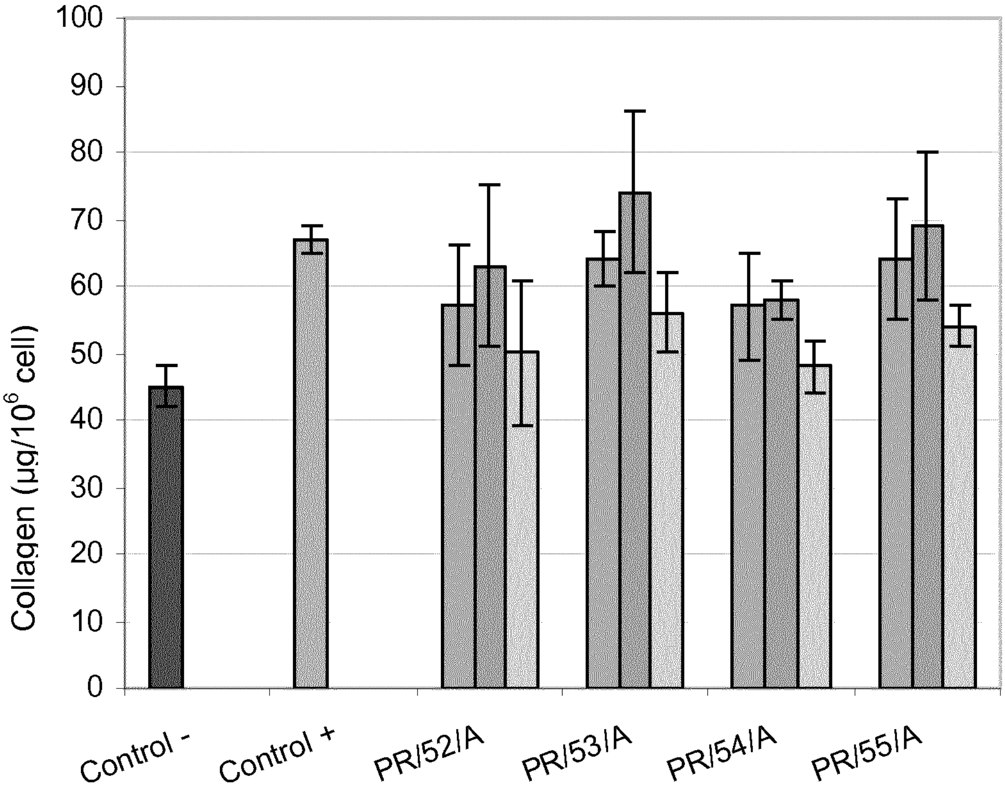

100
90
80
70
60
50
40
30
20
10
0
Collagen (µg/10^6 cell)
Control -
Control +
PR/52/A
PR/53/A
PR/54/A
PR/55/A


**FIG. 1**

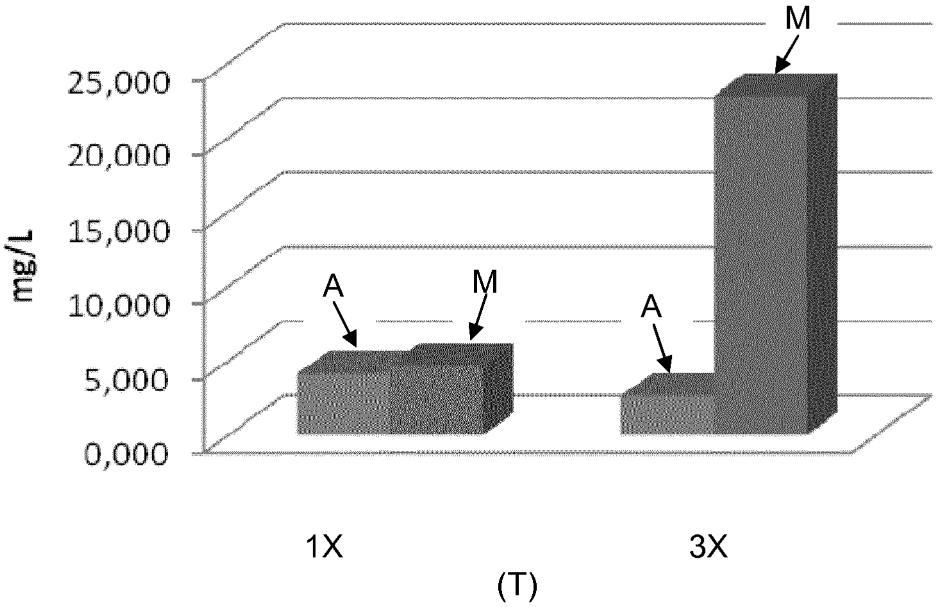


**FIG. 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 867447 A
- EP 200747EC A


### Non-patent literature cited in the description


- **MAQUART et al.** Stimulation of collagen synthesis in fibroblast cultures by a triterpene extracted from Centella asiatica. *Conn. Tissue Res,* 1990, vol. 24, 107-120
- **BONFILL et al.** *Plant cell, tissue and organ culture,* 2010, vol. 104 (1), 61-67
- **MULABAGAL et al.** Plant cell cultures-an alternative and efficient source for the production of biologically important secondary metabolites. *International Journal of Applied Science, Engineering and Technology,* 2004, vol. 2, 101-153
- **BONFILL et al.** Production of centellosides and phytosterols in cell suspension cultures of Centella asiatica. *Plant Cell Tiss Organ Cult,* 2011, vol. 104, 61-67
- **MULABAGAL et al.** Plant cell cultures-an alternative and efficient source for the production of biologically important secondary metabolites. *International Journal of Applied Science, Engineering and Technology-,* 2004, vol. 2, 101-153